# EUROPEAN PATENT SPECIFICATION

(11) **EP 0 742 838 B1**
(45) Date of publication and mention of the grant of the patent: **13.08.2003**
(21) Application number: 95909385.7
(22) Date of filing: 30.01.1995
(51) Int. Cl.: C12Q 1/68

(54) **METHODS FOR THE ELIMINATION OF DNA SEQUENCING ARTIFACTS**
METHODE ZUR ELIMINIERUNG VON SEQUENZIERARTEFAKTEN
PROCEDES D'ELIMINATION D'ARTEFACTS DE SEQUENCAGE D'ADN

(30) Priority: 31.01.1994 US 189240
(43) Date of publication of application: 20.11.1996
(73) Proprietor: THE REGENTS OF THE UNIVERSITY OF CALIFORNIA, Oakland, California 94612-3550 (US)
(72) Inventor: CHAMBERLIN, Michael, Kensington, CA 94708 (US); MYTELKA, Daniel, Berkeley, CA 94709 (US)
(74) Representative: Dehmel, Albrecht, Dr.
(86) International application number: US9501200
(87) International publication number: WO95020682

(56) References cited:
- DE-C- 4 411 588
- US-A- 5 545 539
- WINSHIP P R: "AN IMPROVED METHOD FOR DIRECTLY SEQUENCING PCR AMPLIFIED MATERIAL USING DIMETHYL SULPHOXIDE" NUCLEIC ACIDS RESEARCH,GB,OXFORD UNIVERSITY PRESS, SURREY, vol. 17, no. 3, 1989, page 1266 XP000371660 ISSN: 0305-1048
- THAKAR M ET AL: "OSMOLYTE MEDIATION OF T7 DNA POLYMERASE AND PLASMID DNA STABILITY" BIOCHEMISTRY,US,AMERICAN CHEMICAL SOCIETY. EASTON, PA, vol. 33, no. 40, 11 October 1994 (1994-10-11), page 12255-12259 XP002007763 ISSN: 0006-2960
- PROCEEDINGS NATIONAL ACADEMY OF SCIENCE USA, Volume 70, No. 2, issued February 1973, MELCHIOR et al., "Alteration of the Relative Stability of DA-DT and DG-DC Base Pairs in DNA", pages 298-302.
- PROCEEDINGS OF THE NATIONAL ACADEMY OF SCIENCE USA, Volume 74, SANGER et al., "DNA Sequencing with Chain-Terminating Inhibitors", pages 5463-5467.
- BIOTECHNIQUES, Volume 7, Number 5, issued 1989, DEL SAL et al., "The CTAB-DNA Precipitation Method: A Common Mini-Scale Preparation of Template DNA from Phagemids, Phages or Plasmids Suitable for Sequencing", pages 514-519.
- EDITORIAL COMMENT, Volume 19, No. 2, issued 1992, WILLIAMSON et al., "Glycerol Tolerant DNA Sequencing Gels", pages 29-36.
- BIOCHEMISTRY, Volume 32, issued 1993, REES et al., "Betaine can Eliminate the Base Pair Composition Dependence of DNA Melting", pages 137-144.
- BIOCHEMISTRY, Volume 31, issued 1992, SANTORO et al., "Increased Thermal Stability of Proteins in the Presence of Naturally Occurring Osmolytes", pages 5278-5283.

## Description

### BACKGROUND OF THE INVENTION

The present invention relates primarily to improvements in methods of DNA sequencing. In particular, the invention relates primarily to the elimination of stops or pauses in chain termination methods of DNA sequencing by the addition of nitrogen-containing organic compounds such as betaine, trimethylamine N-oxide and dimethylglycine. The invention also provides for DNA sequencing kits containing these compounds. The invention also provides for improvements in other laboratory procedures using DNA polymerases, such as polymerase chain reaction (PCR).

Efficient DNA sequencing technology is very important to the development of the biotechnology industry as well as for basic biological research. Improvements in both efficiency and accuracy of DNA sequencing are needed to keep pace with the demands for DNA sequence information. The Human Genome Project, for example, has set a goal for dramatically increasing the efficiency, cost-effectiveness and throughput of DNA sequencing techniques. (See Collins, F., and Galas, D. (1993) Science 262:43-46.)

Most DNA sequencing today is carried out by a chain termination method of DNA sequencing. The most popular chain termination methods are variants of the dideoxynucleotide-mediated chain termination method of Sanger (see Sanger *et al*. (1977) *Proc. Nat. Acad. Sci., USA* 74:5463-5467). Thousands of laboratories employ this technique including those doing automated sequencing for the Human Genome Project. Commercial kits containing the reagents needed for this method of DNA sequencing are available and are widely used.

Although commonly used, the Sanger (dideoxy) sequencing technique has problems and limitations. One of the major problems with this method is the incidence of DNA polymerase stops or pauses which interfere with the determination of the DNA sequence. Stops are predominantly problematic in regions of the DNA that are GC-rich or in regions that are especially far from the primer. In addition, stops occur more frequently in impure DNA preparations. Because of this, DNA purification is generally required before DNA can be sequenced by the dideoxy method.

Various methods have been proposed to eliminate stops in dideoxy sequencing. For example, researchers have tried varying the reaction temperature, using a variety of DNA polymerases, stabilizing the DNA polymerase, and extending the prematurely terminated DNA molecules with terminal deoxynucleotidyl transferase (see T.W. Fawcette and S.G. Bartlett (1990) *BioTechniques* 9:46-48; D. Pisa-Williamson and C.W. Fuller (1992) *United States Biochemical Corp. Comments* 19:29-36; J. Sambrook, E.F. Fritsch and T. Maniatis, ed. (1989) *Molecular Cloning: A Laboratory Manual*, second edition, Cold Spring Harbor Press, Cold Spring Harbor, N.Y.; and F.M. Ausubel, R. Brent, R.E. Kingston, D.D. Moore, J.G. Seidman, J.A. Smith and K. Struhl, ed. (1989) *Current Protocols in Molecular Biology,* Greene Publishing Associates and Wiley-Interscientific, John Wiley and Sons, New York.) However, none of these methods has been reliable. There is a continuing need to eliminate the problem of stops in DNA sequencing and thereby improve the efficiency and cost-effectiveness of this important process.

### SUMMARY OF THE INVENTION

This invention provides for methods of decreasing the incidence of DNA polymerase stops that occur in a reaction mixture containing a DNA polymerase. The incidence of DNA polymerase stops is decreased by the addition of nitrogen-containing organic molecules, which are described herein, to the reaction mixture. These compositions of the invention are added to the reaction mixture in an amount that is effective to decrease the incidence of DNA polymerase stops. The reaction mixture can be used in a chain termination method of DNA sequencing. Preferably the chain termination method of DNA sequencing is a dideoxynucleotide DNA sequencing method. The reaction mixture can also be a PCR reaction mixture. The DNA which is sequenced or amplified can be purified or unpurified. Unpurified DNA can be present, for example, as a crude cell lysate.

The method of decreasing the incidence of DNA polymerase stops can be combining, in an aqueous solution, a DNA molecule, a DNA polymerase or DNA polymerases, a mixture of deoxyribonucleoside triphosphates, a chain elongation inhibitor, and one or more of the compositions of the invention to form a reaction mixture. The DNA polymerase is capable of producing a nucleic acid complementary to a portion of the DNA molecule by using the DNA molecule as a template. The reaction mixture is incubated to allow the DNA polymerase to form nucleic acid fragments of varying length by using the DNA molecule as a template. The nucleic acid fragments that are formed are complementary to the DNA molecule that is being sequenced.

In some variations of the above method, a nucleic acid primer is also added to the reaction mixture. This primer is complementary to a second portion of one strand of the DNA molecule that is located downstream from the first portion of that strand. The DNA polymerase that is used is capable of extending the 3' end of the primer to produce a nucleic acid that is complementary to the first portion of the DNA molecule.

The invention provides improved methods for sequencing a DNA molecule by the chain termination method, wherein the DNA molecule is combined with a DNA polymerase capable of producing a nucleic acid complementary to a portion of the DNA molecule by using the DNA molecule as a template; with a mixture of deoxyribonucleoside triphosphates; and with chain elongation inhibitor, to form a reaction mixture. This reaction mixture is incubated to permit the DNA polymerase to form nucleic acid fragments of varying length by using the DNA molecule as a template, wherein the nucleic acid fragments are complementary to the DNA molecule. The improved part of this method is the addition to the reaction mixture of an amount of one or more of the nitrogen-containing organic compounds described herein.

The invention also provides for kits for DNA sequencing by the chain termination method. These kits have instructional material, a container which contains one or more deoxyribonucleoside triphosphates, a container which contains a chain elongation inhibitor, and an amount of the compositions of the invention. The kits may also contain other components. Preferably, the kits are for DNA sequencing by the dideoxy DNA sequencing method.

### BRIEF DESCRIPTION OF THE DRAWINGS

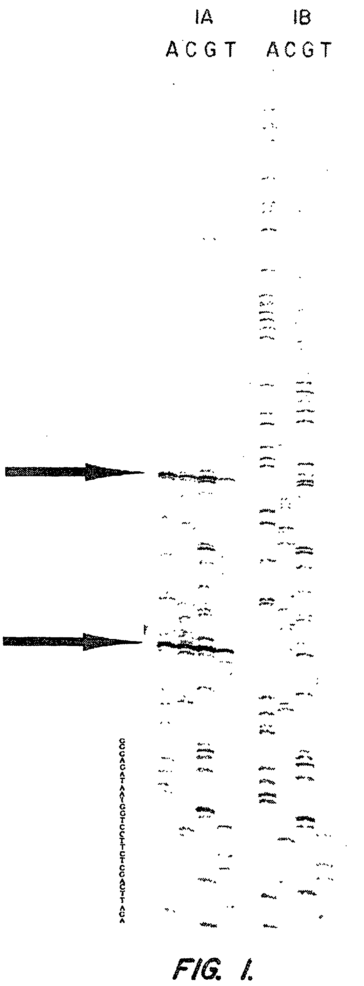

Figure 1A. DNA polymerase stops in dideoxy DNA sequencing. Dideoxy DNA sequencing of double-stranded DNA was carried out as described in example 1 herein, except that water was substituted for betaine (results were identical with or without water). The reaction mixture was separated on a polyacrylamide/urea DNA sequencing gel as described in example 1. The positions of DNA polymerase stops are shown by arrows.
Figure 1B. Elimination of DNA polymerase stops in dideoxy DNA sequencing by betaine. Dideoxy DNA sequencing was carried out in the presence of betaine as described in example 1. The reaction mixture was separated on a polyacrylamide/urea DNA sequencing gel as described in example 1. The arrows show the positions corresponding to the DNA polymerase stops of figure 1A.

### DEFINITIONS

The term "nucleic acids", as used herein, refers to either DNA or RNA. It includes plasmids, infectious polymers of DNA and/or RNA, nonfunctional DNA or RNA, chromosomal DNA or RNA and DNA or RNA synthesized *in vitro* (such as by the polymerase chain reaction). "Nucleic acid sequence" or "polynucleotide sequence" refers to a single- or double-stranded polymer of deoxyribonucleotide or ribonucleotide bases read from the 5' to the 3' end.

The term "DNA molecule" as used herein refers to DNA molecules in any form, including naturally occurring, recombinant, or synthetic DNA molecules. The term includes plasmids, bacterial and viral DNA as well as chromosomal DNA. The term encompasses DNA fragments produced by cell lysis or subsequent manipulation of DNA molecules. Unless specified otherwise, the left hand end of single-stranded DNA sequences is the 5' end.

The term "upstream" when used in respect to a single stranded DNA molecule, refers to those DNA sequences located 5' to a reference region of the DNA molecule. For example, those sequences located upstream from a defined portion of a DNA molecule are those located 5' to the defined portion. Similarly, the term "downstream", when used with respect to single stranded DNA molecule, refers to those DNA sequences located 3' to a reference region of the DNA molecule.

The term "complementary" as used herein refers to a relationship between two nucleic acid sequences. One nucleic acid sequence is complementary to a second nucleic acid sequence if it is capable of forming a duplex with the second nucleic acid, wherein each residue of the duplex forms a guanosine-cytidine (G-C) or adenosine-thymidine (A-T) basepair or an equivalent basepair. Equivalent basepairs can include nucleoside or nucleotide analogues other than guanosine, cytidine, adenosine, or thymidine, which are capable of being incorporated into a nucleic acid by a DNA or RNA polymerase on a DNA template. A complementary DNA sequence can be predicted from a known sequence by the normal basepairing rules of the DNA double helix (see Watson J.D., *et al*. (1987) *Molecular Biology of the Gene*, Fourth Edition, Benjamin Cummings Publishing Company, Menlo Park, California, pp. 65-93). Complementary nucleic acids may be of different sizes. For example, a smaller nucleic acid may be complementary to a portion of a larger nucleic acid.

The term "DNA template" or "template" as used herein, refers to a DNA molecule or portion of a DNA molecule that is used by a DNA or RNA polymerase to determine the sequence of a newly synthesized nucleic acid. (See Watson, J.D., *et al., supra* for a detailed description of the action of DNA and RNA polymerases on template DNA molecules).

The term "replicating", as used herein in reference to a DNA polymerase, refers to the process of using a DNA molecule as a template to produce a nucleic acid complementary to a portion of the DNA molecule.

The terms "purified DNA" or "purified DNA molecule," as used herein, refers to DNA that is not contaminated by other biological macromolecules, such as RNA or proteins, or by cellular metabolites. Purified DNA contains less than 5% contamination (by weight) from protein, other cellular nucleic acids and cellular metabolites. The terms "unpurified DNA" or "unpurified DNA molecules" refer to preparations of DNA that have greater than 5% contamination from other cellular nucleic acids, cellular proteins and cellular metabolites. Unpurified DNA may be obtained by using a single purification step, such as precipitation with ethanol combined with either LiCl or polyethylene glycol. The term "crude cell lysate preparation" or "crude cell lysate" or "crude lysate" refers to an unpurified DNA preparation where cells or viral particles have been lysed but where there has been no further purification of the DNA.

Some compounds of the invention may be present with a positive or negative charge or with both a positive and negative charges, depending on the pH of the solution. It is understood that these various forms of these compounds are included in the present invention.

The term betaine, as used herein, refers to N,N,N-trimethylglycine.

All numerical ranges in this specification are intended to be inclusive of their upper and lower limits.

### DETAILED DESCRIPTION

### A. Introduction

There are a number of different methods of determining the sequence of DNA molecules. Most methods in common use today are variations on one of two general methods. One general scheme uses chemical reagents which react with specific bases to allow other chemicals to cleave the phosphodiester backbone at those points. The second general method uses a polymerase enzyme to produce DNA fragments complementary to the DNA molecule to be sequenced. The inclusion of low amounts of chain elongation inhibitors in the reaction mixture causes different sized DNA fragments to be generated. The DNA fragments generated by either chemical methods or chain termination methods can be separated and analyzed by a variety of methods. For example, they can be separated on a sequencing gel based on size. By examining the bands present at any position on the gel, the sequence of the DNA molecule can be determined at the corresponding position. For example, the top of the sequence noted in Figure 1A contains three consecutive G nucleotides, since bands are present at that position in only the G lane.

Chain termination methods of DNA sequencing are much more widely used than chemical cleavage methods. Dideoxynucleoside triphosphates are the most common chain terminators used in DNA sequencing. For examples of specific protocols for dideoxy DNA sequencing, see Sambrook, *et al*., *supra*.

In spite of its wide use, there are technical problems associated with the dideoxy sequencing method. One of the major problems is the incidence of stops or pauses of the DNA polymerase which interfere with the determination of the DNA sequence. When the DNA polymerase stops at a particular site, bands appear in all four lanes of the sequencing gel, and the nucleotide residue at that position cannot be determined.

DNA polymerase stops occur for several reasons. Stops are observed more frequently as the enzyme extends farther from the 3' end of the primer. This phenomenon limits the length of DNA fragment that can sequenced at one time. Reducing the incidence of stops allows longer pieces of DNA to be sequenced and therefore makes DNA sequencing more efficient.

DNA polymerase stops also occur more frequently when contaminants are present in the DNA preparation. Because of this phenomenon, DNA preparations must be purified prior to sequencing. The improved DNA sequencing method of the invention decreases the incidence of stops and therefore allows less pure preparations of DNA to be reliably sequenced. This, in turn, increases the efficiency and reduces the cost of DNA sequencing.

Lastly, even under optimal conditions, DNA polymerase stops are often seen in GC-rich regions of the DNA. This makes DNA sequence information in certain areas of a DNA molecule difficult to determine. This problem is, of course, compounded when one is trying to determine a DNA sequence in a GC-rich region far from the 3' end of the primer or in a DNA preparation that has not been optimally purified. Thus, the improved method of the invention increases the accuracy and efficiency of DNA sequencing while reducing its cost.

The reduced incidence of DNA polymerase stops caused by the compounds of the invention also has utility in procedures other than DNA sequencing. Numerous common laboratory techniques, such as PCR (polymerase chain reaction), *in vitro* mutagenesis, nick translation, reverse transcription and blunt ending utilize DNA polymerases. The addition of appropriate concentrations of the compounds of the invention can potentially increase the efficiency or speed of these procedures.

### B. Compounds that reduce the incidence of DNA polymerase stops

The compounds used in the present invention are nitrogen-containing organic molecules that are capable of eliminating or reducing the incidence of stops occurring in chain-termination methods of DNA sequencing and in other laboratory procedures using DNA polymerases, such as PCR. These compounds are represented by the formula: 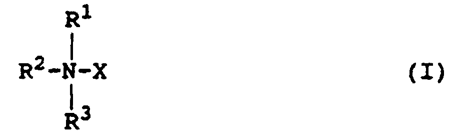 wherein:
R¹, R², and R³ may be the same or different and are independently selected from the group consisting of hydrogen, methyl, ethyl, and propyl, with the proviso that (i) no more than two of R¹, R², and R³ are hydrogen and (ii) R¹ is ethyl or propyl when combined with R⁴ to form a pyrrolidine ring; and
X is either
   (a) =O ; or
   (b) 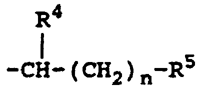
wherein:
R⁴ is selected from the group consisting of methyl, hydrogen and, when combined with R^{1,} forms a pyrrolidine ring;
R⁵ is selected from the group consisting of -CO₂H and -SO₃H; and
n is an integer of from 0 to 2; and
with the proviso that, when R¹ and R⁴ form a pyrrolidine ring, no more than one of R² and R³ is hydrogen, and wherein the composition is added in an amount effective to decrease the incidence of stops.

When a pyrrolidine ring is formed by R¹ and R^{4,} a compound of formula II is formed.

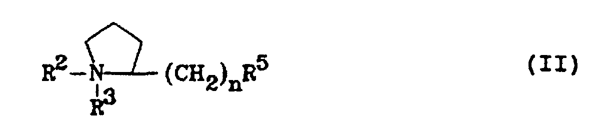

In certain preferred embodiments, the methods and kits of this invention use compounds of formula I wherein R¹, R² and R³ are the same or different and selected from the group consisting of methyl, ethyl and hydrogen with the proviso that no more than two of R¹, R² and R³ are hydrogen and, when R¹ and R⁴ form a pyrrolidine ring, no more than one of R² and R³ are hydrogen.

In another group of preferred embodiments, the methods and kits of this invention use a compound of formula I wherein X is -CH₂CO₂H. Further preferred embodiments within this group use compounds where R¹, R² and R³ are methyl; where R¹, R² are methyl and R³ is hydrogen; or where R¹ is methyl and R² and R³ are hydrogen.

In further preferred embodiments, the methods and kits of this invention use a compound of formula I wherein X is =O and R¹, R² and R³ are methyl.

In still further preferred embodiments, the methods and kits of this invention use a compound of formula I wherein R¹ and R⁴ form a pyrrolidine ring, R² and R³ are methyl, n is 0, and R⁵ is -CO₂H (stachydrine, formula III).

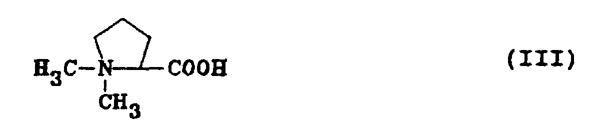

In yet another group of preferred embodiments, the methods and kits of this invention use compounds wherein R¹, R², and R³ are methyl and X is -CH₂-SO₃H (sulfobetaine).

In general, the compounds used to eliminate or reduce the incidence of DNA polymerase stops in DNA sequencing and in other laboratory procedures using a DNA polymerase are commercially available. For example, betaine, dimethylglycine, sarcosine, and trimethylamine N-oxide can all be obtained from Sigma Chemical Company (St. Louis, Missouri, USA).

These compounds may also be synthesized by routine methods known to those of skill in the art. For example, compounds of formula wherein R⁴ is H, n is 0 and R⁵ is -CO₂H can be synthesized by the method of Lloyd, *et al.* (1992) *J. Pharm. Pharmacol*. 44: 507-511. In general, ethyl chloroacetate is heated to reflux with the appropriate tertiary amine in ethanol. When the reaction is complete, the ethanol is removed from the reaction mixture by evaporation under reduced pressure. The residue is dissolved in 3-6% w/v aqueous HCl and warmed to reflux. Evaporation of the solvent under reduced pressure provides the desired products. Typically, these products can be recrystallized from an acetonitrile/water mixture.

Compounds of formula I wherein R⁴ is H or CH₃, n is 1 and R⁵ is CO₂H can be synthesized by the method of Fiedorek, F.T., U.S. Patent No. 2,548,428. In brief, betalactones are reacted with tertiary amines to provide the desired compounds.

Compounds of formula I wherein R⁴ is H, n is 2, and R⁵ is -CO₂H can be synthesized by the method of Aksnes, G., et *al. J. Chem. Soc. London* 1959:103-107. In brief, 4-bromobutyric acid (Aldrich Chemical Co., Milwaukee, Wisconsin, USA) is converted to a methyl ester by treatment with methyl alcohol and catalytic sulfuric acid. Subsequent treatment of the methyl ester with excess alcoholic tertiary amine provides the desired compounds.

Compounds of formula I wherein R⁴ and R¹ are taken together to form a pyrrolidine ring and where R⁵ is CO₂H are synthesized by the general method of Karer, *et al*. (1925) *Helv. Chim. Acta*. 8: 364. For example, stachydrine is formed by the methylation of proline, according to this procedure.

Compounds of formula I wherein X is =O are synthesized by oxidation of the corresponding tertiary amines (see March, J. (1992) *Advanced Organic Chemistry, Reactions, Mechanisms and Structure, Fourth Edition,* John Wiley and Sons, New York, pp. 1200-1201). Typically, the oxidation is carried out with hydrogen peroxide, but other peracids may also be used.

Sulfobetaine can be synthesized according to the procedure of King, J.F., et al. (1985) *J. Phosphorus Sulfur* 25: 11-20. Other compounds of formula I wherein R⁵ is -SO₃H can also be synthesized by modifications of this procedure and by other methods known to those of skill in the art.

### C. Improved methods for DNA sequencing, polymerase chain reaction and other laboratory procedures using DNA polymerases

### 1. Chain termination methods of DNA sequencing

The present invention encompasses an improvement in DNA sequencing by the chain termination method. As used herein, the terms "chain termination method of DNA sequencing," "chain termination method" or "chain termination DNA sequencing method" refer to a DNA sequencing method that uses a DNA polymerase to produce nucleic acid fragments complementary to a portion of the DNA molecule to be sequenced. Generally, a primer is used that is complementary to a portion of the DNA molecule. The primer is extended along the DNA molecule template by the DNA polymerase. The principle of the method is that low amounts of specific chain elongation inhibitors are included in the reaction mixture so that the DNA polymerase will only occasionally incorporate an inhibitor and terminate. Generally, four reaction mixtures are set up, each with a different chain elongation inhibitor, capable of specifically terminating at a guanosine, cytosine, adenosine or thymidine residue. The DNA fragments generated by incubation with the DNA polymerase can be separated and analyzed to determine the sequence of the DNA molecule. Typically, the fragments are separated by gel electrophoresis and detected by autoradiography, although other separation or detection methods may also be used. (See Sanger, et al., *supra* and Sambrook, *et al., supra,* for a more detailed description of the chain termination method of DNA sequencing.) This procedure is amenable to automation, and various specific methods have been created for that purpose, including single lane sequencing, laser detection and capillary electrophoresis.

The terms "chain elongation inhibitors" or "chain-terminating inhibitors", as used herein, refer to compounds that terminate nucleic acid chain elongation by a DNA polymerase enzyme. As described above, these compounds may be useful in chain termination methods of DNA sequencing.

The terms "pause", "stop", "DNA polymerase pause" or "DNA polymerase stop" as used herein refer to a phenomenon wherein the DNA polymerase stops or does not function at a particular nucleotide when the DNA polymerase is incubated with DNA in a chain terminating DNA sequencing method. This is a phenomenon in which DNA polymerase molecules fail to continue elongation despite the presence of the next nucleotide to be incorporated and the absence of any manifest reason why elongation should cease (such as the incorporation of a dideoxynucleotide). When the DNA fragments are displayed on a DNA sequencing gel, stops appear as bands in all four lanes of the gel at a position corresponding to the length of the fragment that the DNA polymerase molecules had difficulty elongating, so the identity of the nucleotide at that position cannot be determined (see arrows in Figure 1A for examples).

There are a variety of different chain termination methods for sequencing DNA. The most commonly used method is the dideoxynucleotide DNA sequencing method. The terms "dideoxynucleotide DNA sequencing method" or "dideoxy DNA sequencing method", as used herein, refer to a chain termination method of DNA sequencing wherein the chain elongation inhibitors are 2'3'-dideoxynucleosides or their derivatives. Typically, 2'3'-dideoxynucleoside triphosphates are used. A variety of other chain elongation inhibitors may also be used. For example, arabinonucleoside derivatives or 3' O-methyl deoxyribonucleotide derivatives may be used in chain termination methods. (See Sanger, *et al., supra* and Axelrod, V.O., *et al*. (1978) *N.A.R.* 5:3549-3563.)

A variety of different enzymes can be used to produce DNA fragments complementary to the DNA molecule to be sequenced. For example, T7 DNA polymerase, Taq polymerase, and the Klenow fragment of DNA polymerase I are all used in DNA sequencing. RNA polymerase and reverse transcriptase have also been used. DNA polymerases may be genetically or physically altered to optimize the enzyme for use in DNA sequencing.

The DNA polymerases require a primer sequence. The primer sequences may be generated by digestion of the DNA molecule or may be added exogenously as a primer molecule. The terms "primer molecule", "primer" or "nucleic acid primer" or "DNA primer", as used herein, refer to a single-stranded nucleic acid molecule which is complementary to a portion of the DNA molecule to be sequenced. Primers are allowed to anneal to a DNA molecule, so that subsequent elongation of the 3' end of the primer by a DNA polymerase produces a nucleic acid sequence complementary to a portion of the DNA molecule to be sequenced. Primers are commonly used in chain termination methods of DNA sequencing. However, in certain modifications of DNA sequencing by the chain termination method, primer sequences may be generated by digestion with appropriate enzymes (see below).

In an alternative embodiment, DNA sequencing vectors may be used to facilitate DNA sequencing by chain termination methods. A variety of such vectors may be used, including M13, Bluescript vectors such as pBS-KS⁺ and pBS-KS⁻ (Stratagene, San Diego, California, U.S.A.), pUC vectors such as pUC18 and pUC19 and pBR322 vectors. The DNA molecule to be sequenced is inserted into the sequencing vector. Primers can be constructed that are complementary to a region of the DNA in the sequencing vector, so that elongation from the 3' end of the primer will produce DNA fragments complementary to portions of the DNA molecule to be sequenced. Thus, when sequencing vectors are used, the DNA molecule to be sequenced can be present as an insert in the vector. Primers that are complementary to a region of the DNA sequencing vector can be used, so that chain elongation occurs from the 3' end of the primer into the region of the DNA insert, thereby allowing determination of the DNA sequence of the insert.

The DNA preparation for sequencing may be either purified or unpurified. Unpurified DNA may be a crude cell lysate. The compounds of the invention that eliminate or reduce the incidence of DNA polymerase stops allow for the use in DNA sequencing of unpurified DNA preparations, including crude cell lysates.

The DNA preparation may be present in single-stranded or double-stranded form. For instance, linear double-stranded DNA may be generated by PCR. When such DNA is sequenced, the incidence of DNA polymerase stops is reduced or eliminated.

The mixture of DNA fragments generated in chain termination methods of DNA sequencing is most commonly separated and analyzed by gel electrophoresis. See figure 1A and 1B herein for an example of a DNA sequencing gel. Other separation methods useful for separating DNA fragments may also be used (see Sambrook, *et al*., supra). For instance, mass spectrometry can be used to analyze mixtures of DNA fragments (see Jacobson, *et al.* (1991) *J. Genetic Analysis, Techniques and Applications* 8:223-229.

The DNA fragments may be labeled or unlabeled. For example, unlabeled DNA fragments may be detected by ultraviolet spectroscopy in an automated technique or by silver staining after gel electrophoresis. However, it is generally desirable to produce labeled DNA fragments to facilitate their detection. For instance, radiolabeled DNA fragments may be produced. They may be produced, for example, by use of a radioactive primer. Alternatively, radioactive nucleotides may be incorporated during the incubation with a DNA polymerase. When radiolabeled DNA fragments are produced, they may be separated by gel electrophoresis and visualized by autoradiography (see figures 1A and 1B, herein). A wide variety of non-radioactive labels may also be used including fluorophores, chemiluminescent agents and small detectable molecules, such as biotin. For example, in automated sequencing techniques, fluorescent or colorimetric labels can be detected by laser spectroscopy using capillary tubes or sequencing gels.

Non-radioactive labels may be attached by indirect means. Generally, a ligand molecule (*e.g.*, biotin) is covalently bound to the molecule. The ligand then binds to an anti-ligand (*e.g.*, streptavidin) molecule which is either inherently detectable or covalently bound to a signal system, such as a detectable enzyme, a fluorescent compound, or a chemiluminescent compound. A number of ligands and anti-ligands can be used. Where a ligand has a natural anti-ligand, for example, biotin, thyroxine, and cortisol, it can be used in conjunction with the labelled, naturally occurring anti-ligands. Alternatively, any haptenic or antigenic compound can be used in combination with an antibody.

There are a variety of modifications of the chain termination method of DNA sequencing. For example, DNA sequencing may be performed manually, by semi-automated procedures or by the use of an automated DNA sequencing instrument. In addition, chain termination DNA sequencing technology may be combined with other techniques. For example, in "exometh" methods, a DNA molecule is treated with exonuclease to generate a series of DNA molecules with single-stranded 5' extensions of varying length. These DNA molecules can then be sequenced directly by a chain termination method of DNA sequencing without the addition of an exogenous primer. (See Sorge, J.A., *et al*. (1989) *Proc. Nat. Acad. Sci, USA* 86: 9208-9212 for a more detailed description of DNA sequencing by the "exometh" modification.) Such modifications of chain termination DNA sequencing methods are all encompassed by the present invention.

The compounds of the invention may be added to the reaction mixture before or during the incubation of the DNA with the DNA polymerase to eliminate or reduce the incidence of stops. The order of addition of the DNA molecule, the DNA polymerase, the chain elongation inhibitors, the deoxyribonucleoside triphosphates, the primer, and the compounds of the invention may also be varied. As demonstrated in Example 4, the compounds that reduce or eliminate the incidence of stops may even be added after the normal incubation time for the DNA polymerase, and the incubation may be extended to remove DNA polymerase stops.

### 2. Polymerase chain reaction procedures

The present invention also encompasses an improvement in nucleic acid amplification procedures, such as PCR, which involve chain elongation by a DNA polymerase.

There are a variety of different PCR techniques which utilize DNA polymerase enzymes, such as Taq polymerase. See *PCR Protocols: A Guide to Methods and Applications.* (Innis, M, Gelfand, D., Sninsky, J. and White, T., eds.), Academic Press, San Diego (1990) for detailed description of PCR methodology. In a typical PCR protocol, a target nucleic acid, two oligonucleotide primers (one of which anneals to each strand), nucleotides, polymerase and appropriate salts are mixed and the temperature is cycled to allow the primers to anneal to the template, the polymerase to elongate the primer, and the template strand to separate from the newly synthesized strand. Subsequent rounds of temperature cycling allow exponential amplification of the region between the primers. The primers can anneal to both the original template and the newly synthesized nucleic acid, as long as the polymerase is able to extend at least as far as the position to which the other primer anneals. For this reason, the ability of PCR to amplify a product is primarily limited by the ability of the polymerase to extend the annealed primer. The ability of the polymerase to extend the annealed primer is dependent on the distance between the primers and the nucleotide composition of the sequence between them. The addition of an appropriate amount of one or more of the compounds described herein facilitates this elongation. See example 8 and example 9, herein, for a demonstration of the effects of betaine on chain elongation in PCR protocols using Taq polymerase. Other compounds of the invention in addition to betaine can be used in a similar manner to improve chain elongation in a variety of different PCR methods.

The compounds of the invention also have utility in reducing DNA polymerase stops in laboratory procedures other than DNA sequencing and polymerase chain reactions. Numerous common laboratory techniques, such as *in vitro* mutagenesis, nick translation, reverse transcription and blunt ending utilize DNA polymerases. See Sambrook, *et al.* (1989) *Molecular Cloning: A Laboratory Manual*, second edition, Vol. 1-3, Cold Spring Harbor Press, Cold Spring Harbor, N.Y. (1989) for a description of these methods. Thus, the compounds of the invention are useful in a variety of procedures involving chain elongation by a DNA polymerase.

### 3. Testing and optimization of compounds that reduce or eliminate the incidence of stops

Compounds capable of eliminating or reducing the incidence of stops in chain termination methods of DNA sequencing and in other laboratory procedures using DNA polymerase are described above. These compounds may be tested for their relative ability to eliminate or reduce DNA polymerase stops. For instance, the compounds may be tested by the procedure described in Example 1, herein. Briefly, DNA sequencing is carried out by a dideoxy sequencing method in the presence or absence of a selected concentration of a compound of the invention. The DNA sequencing gels obtained in the presence and absence of the compound are then compared to determine the effectiveness of the compound in eliminating DNA polymerase stops (see for example, figure 1A and 1B). Effective concentrations for each of the compounds may be determined by this procedure. Optimal concentrations for a given compound may vary for the different variations of chain termination DNA sequencing methods. These concentrations may be readily determined experimentally by adding different amounts of a compound and determining the incidence of stops (see for instance, Example 7, herein).

This invention also encompasses kits for DNA sequencing by the chain termination method which comprise an amount of one or more of the compounds described herein that eliminate or reduce the incidence of DNA polymerase stops. The kits may further comprise instructional material, a container which contains one or more deoxyribonucleoside triphosphates, a container which contains a DNA polymerase, and a container that contains a chain elongation inhibitor. The containers in the kit may be combined in various ways. For example, a chain elongation inhibitor may be combined in the same container with the mixture of deoxyribonucleoside triphosphates.

This invention also encompasses kits for DNA polymerase chain reaction which comprise an amount of one or more of the compounds described herein that eliminate or reduce the incidence of DNA polymerase stops. The kits may further comprise instructional material, a container which contains a DNA polymerase, and a container that contains deoxyribonucleoside triphosphates.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described. Unless mentioned otherwise, the techniques employed or contemplated herein are standard methodologies well known to one of ordinary skill in the art. The materials, methods and examples are illustrative only and not limiting.

### EXAMPLES

### Example 1 Elimination of stops in a dideoxy method of DNA sequencing by betaine

Betaine eliminates stops that are routinely observed in a standard dideoxy sequencing procedure.

Betaine monohydrate was obtained from Sigma and stored as a 5.5M stock solution at -20°C. Modified T7 polymerase (Sequenase 2.0 (Amersham, Arlington Heights, Illinois, U.S.A.)) and nucleotide mixes were purchased from USB. ³⁵S-dATP was purchased from Amersham.

DNA sequencing generally followed the protocol of Del Sal *et al*. (1989) *BioTechniques* 7:514-519. Sequencing was performed by mixing 1 picomole (pmole) of supercoiled double stranded plasmid DNA (purified by running over a QIAGEN tip-100 column according to the manufacturer's specifications) with 2-4 pmole of various oligonucleotide primers (generally purified by polyacrylamide gel electrophoresis, though this proved unnecessary for primers under 30 nucleotides) and adjusting the volume to 10 µl containing 0.1 N NaOH. The tube was incubated for ten minutes at 68°C, then it was moved to room temperature and 4 µl of TDMN (200 mM NaCl, 50 mM DTT, 120 mM HCl, 80 mM MgCl₂, 280 mM TES) were added. After a further ten minute incubation at room temperature, 2 µl of GTP labeling mix (7.5 µM dCTP, 7.5 µM dGTP, 7.5 µM dTTP) and 5 µCi of ³⁵5-dATP were added, followed by 2 µl of Sequenase (diluted 1:8 in cold 10 mM Tris.Cl pH 8.0, 1 mM EDTA). This mix was incubated for five minutes at room temperature, then 3.5 µl were aliquoted to each of four tubes preheated to 37°C, each containing 3.5 µl of 5.5 M betaine and 2.5 µl of one of the termination mixes (80 µM each dNTP, 8 µM one ddNTP, 50 mM NaCl), for a final concentration of approximately 2M betaine. This was incubated for five minutes at 37°C, then stopped with 4 µl of stop solution (80% deionized formamide, 1X TBE, 0.05% xylene cyanol, 0.05% bromophenol blue.) After 2.5 minutes at 95°C, the tubes were briefly chilled on ice. 4.5 µl were loaded on a 6% polyacrylamide (19:1 acrylamide:
bisacrylamide)/7M urea/1X TBE gel, which was run for various periods at 35W. The gels were fixed with a 12% methanol/12% acetic acid solution, transferred to Whatman paper, and dried. They were generally exposed overnight on Kodak XAR 5 film.

The addition of betaine at a concentration of 2M eliminated virtually all stops when DNA sequencing was performed using a modified form of the method of Del Sal *et al., supra,* as described above. Sequencing without betaine leads to occasional stops, which tend to occur in GC-rich regions and in regions particularly far from the primer (Fig. 1A). An analysis of seventeen such stops indicates that they tend to occur after the middle position of sequences similar to pyrimidine-guanine-cytosine, with eleven of seventeen stops examined matching this consensus and the other six differing at only one position. There was no obvious correlation with potential secondary structure of the single stranded template, as determined by the program MFOLD, part of the GCG package. In the presence of betaine (Fig. 1B), all of these stops disappear, allowing the correct sequence to be determined.

### Example 2 Readable DNA sequences farther from a primer are obtained in the presence of betaine

The incidence of stops in DNA sequencing by the dideoxy method increases in regions of the DNA located farther from the primer. This phenomenon limits the useful DNA sequence that can be obtained from one primer. However, the inclusion of betaine in the dideoxy sequencing reaction mixture decreases the incidence of stops in DNA regions far downstream from the primer. This allows useful sequence information to be obtained in these regions of the DNA without doing an additional sequencing reaction with another primer.

DNA sequencing was performed in the presence and absence of 2M betaine as described in example 1. The resulting sequencing reactions were run on a DNA sequencing gel as described in example 1, except that the gel was run for 12 hours to examine longer DNA fragments. These longer DNA fragments clearly showed the sequence of DNA regions farther from the primer than those routinely obtained in dideoxy DNA sequencing procedures. Little useful sequence information could be determined from these gels in the absence of betaine. However, in the presence of betaine, DNA sequence could be read up to at least 520 nucleotides from the 3' end of the primer, with sequences beyond that becoming less readable because of the resolving power of the gel.

### Example 3 DNA sequence determination from unpurified DNA preparations in the presence of betaine

DNA was prepared generally following a standard alkaline lysis technique (See Silhavy *et al*. (1984) *Experiments With Gene Fusions,* Cold Spring Harbor Laboratory, Cold Spring Harbor, New York 147-148), followed by an ethanol precipitation. No organic extraction, RNAse treatment or other further purification technique was used. XL1-blue cells (Stratagene, San Diego, California) containing the pDSM3 plasmid were used as a source of the DNA. The DNA was then sequenced as described for example 1. As expected, there were a large number of stops in the absence of betaine. However, the stops present while sequencing this DNA disappeared when betaine was added to the termination reaction.

### Example 4 Elimination of stops during DNA sequencing by chasing with betaine

Betaine is capable of eliminating stops when added after the normal termination reaction. DNA sequencing was carried out as described in example 1, except that no betaine was added with the termination mixture. After the 5 minute incubation at 37°C, either 3.5 µl of 5.5 betaine solution or 3.5 µl of water was added. The incubation was then continued for an additional 5 minutes and was stopped with stop solution as in example 1. Addition of betaine at this point greatly reduced the stops observed in the absence of betaine. Thus, the bands representing stops on the sequencing gels appear to represent halted DNA polymerase complexes. Betaine, added after the normal incubation with the termination mixes, is capable of allowing the halted DNA polymerase complexes to resume elongation. This demonstrates that betaine is not simply acting by stabilizing the DNA polymerase, since its addition at this point would have no effect if that were the case.

### Example 5 Elimination or reduction of the incidence of stops in modified dideoxy DNA sequencing procedures

The DNA sequencing procedure of example 1 was altered to demonstrate that betaine is able to eliminate stops that occur in a variety of dideoxy DNA sequencing protocols. The effect of substituting a single-stranded DNA substrate or using different DNA polymerases in a dideoxy DNA sequencing procedure was examined.

### a) Sequencing of single-stranded DNA

Single stranded DNA was prepared from strain XL1-blue containing plasmid pDSM6, according to routine procedures. When single stranded DNA was sequenced by the procedure described in example 1, fewer stops were observed in the absence of betaine than in double-stranded specimens. However, these stops were also eliminated by betaine.

### b) Sequencing using Klenow DNA polymerase enzyme

DNA sequencing was carried out essentially by the procedure described in example 1, except that the Klenow fragment of *E. coli* DNA polymerase was used. 7.5 units of the Klenow enzyme was used and the extension mix was modified to include 20 µCi of ³⁵S-dATP and 0.1 µl of DTT in addition to the GTP extension mix. Stops were observed in different places with the use of the Klenow enzyme but were generally eliminated by betaine, just as described above for the procedure using Sequenase.

### c) Sequencing using Taq polymerase enzyme

DNA sequencing was carried out according to the Promega "fmol™ DNA Sequencing System" protocol (Promega Corporation, Madison, Wisconsin, USA). The protocol is described below.
(1) End label primer.
(2) Mix 4-40 fmol DNA template, 5 µl 5x fmol sequencing buffer (250 mM Tris.Cl pH 9.0, 10 mM MgCl2), 1.5 pmole labeled primer, betaine and water to a final volume of 16 µl containing an appropriate concentration of betaine (e.g., 2M).
(3) Add 1 µl sequencing Grade Taq DNA Polymerase (5 U/µl)
(4) Add 4 µl of the above to each of the dNTP/ddNTP mixes according to the following table:

| | A | C | G | T |
|---|---|---|---|---|
| dATP | 20 µM | 20 µM | 20 µM | 20 µM |
| dCTP | 20 µM | 20 µM | 20 µM | 20 µM |
| 7 Deaza dGTP | 20 µM | 20 µM | 20 µM | 20 µM |
| dTTP | 20 µM | 20 µM | 20 µM | 20 µM |
| ddATP | 350 µM | - | - | - |
| ddCTP | - | 200 µM | - | - |
| ddGTP | - | - | 30 µM | - |
| ddTTP | - | - | - | 600 µM |

(5) Overlay with one drop of mineral oil.
(6) Place tubes in thermal cycler preheated to 95°C.
(7) Start appropriate cycling program (varies with primer, one example is 95°C for 2 minutes, 30 cycles of (95°C for 30 sec., 42°C/30 sec., 70°/60 sec.)).
(8) Add 3 µl stop solution (10 mM NaOH, 95% formamide, 0.05% bromophenol blue, 0.05% xylene cyanol).
(9) Heat at 95°C for 2½ minutes, ice quench, and load on a sequencing gel.

Sequencing gels were run and the presence of DNA polymerase stops was determined as described in Example 1, herein. The incidence of stops observed in the reaction mixture was reduced in the presence of betaine.

### Example 6 Elimination of stops during DNA sequencing by Dimethylglycine, sarcosine (monomethylglycine) and trimethylamine N-oxide (TMANO)

Three additional compounds were demonstrated to decrease the incidence of stops in a dideoxy DNA sequencing procedure. Dimethylglycine, sarcosine (monomethylglycine) and trimethylamine N-oxide (TMANO) (all from Sigma Chemical Company) were evaluated for their ability to eliminate stops in a dideoxy DNA sequencing method, as described in Example 1. DNA sequencing was otherwise performed as described in example 1. TMANO proved to eliminate stops better than betaine, while dimethylglycine was somewhat less effective. Sarcosine had a measurable effect and is less preferred than the other compounds. Two other N-substituted charged compounds, tetraethylammonium chloride (TEACl) and tetraethylammonium acetate (TEAAc) were also tested. TEACl inhibits the DNA polymerase. TEAAc did not inhibit the DNA polymerase as much as TEACl, but also did not reduce the number of stops observed in the sequencing gel.

### Example 7 Concentration ranges of betaine, TMANO, sarcosine, and dimethylglycine effective in reducing stops in a dideoxy method of DNA synthesis

The concentration ranges of TMANO, sarcosine, dimethylglycine and betaine were determined according to the procedure described in Example 1.

The results are summarized in the following table.

| Compound | Measurable Effect | Maximal Effect |
|---|---|---|
| betaine | 0.5 M | 2 M |
| TMANO | 0.25 M | 0.5 M |
| Dimethylglycine | 0.5 M | 1.5 M |
| Sarcosine | 0.5 M | 1.5 M |

### Example 8 Increasing the maximum size of PCR amplification products

Betaine was shown to extend the maximum distance between primers for chain elongation in PCR. PCR was performed on T7 DNA as described in B. Krummel, Ph.D. Thesis, University of California, Berkeley, 1990. Approximately 50 pmole of each primer were combined with 2.5 Units of AmpliTaq Polymerase (Perkin-Elmer, Norwalk, Connecticut, USA) and 0.5 µg of phage T7 DNA in a 200 µl reaction containing final concentrations of 200 µM dNTP, 50 mM KCl, 10 mM Tris-Cl pH8.3, 2.5 mM MgCl₂ and 0.01% gelatin. Following a 1' hot start at 94°C, 25 cycles of PCR were performed with cycle times of 90" at 94°C, 1' at 37°C, and 20' at 72°C. The products were precipitated, run on a 1% agarose gel and visualized with UV following ethidium staining.

The addition of betaine at a concentration of 2M allowed two primers which were separated by approximately 4000 base pairs to amplify the intervening DNA region. This DNA region was not appreciably amplified in the absence of betaine.

### Example 9 PCR amplification of regions containing problematic DNA sequences

Betaine was shown to allow PCR chain elongation through GC-rich DNA regions which have impeded chain elongation in the absence of betaine. PCR was performed on a plasmid containing multiple repeats of the sequence TGC, which we have shown to impede the progress of DNA polymerases (see Example 1, herein). Although the primers were only 500 base pairs apart, a distance which can normally be easily amplified by PCR, the standard reaction conditions failed to produce any amplified product. When betaine was added to a concentration of 2M, the intervening region was amplified.

## Claims

1. A method of decreasing the incidence of DNA polymerase stops occurring in a reaction mixture containing a DNA polymerase comprising adding to the reaction mixture a compound of the formula: 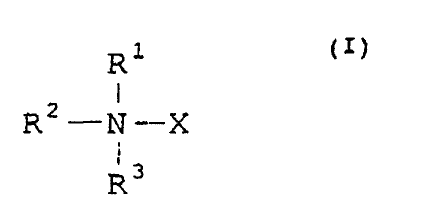 wherein:
R¹, R², and R³ may be the same or different and are independently selected from the group consisting of hydrogen, methyl, ethyl, and propyl, with the provisos that (i) no more than two of R¹, R², and R³ are hydrogen and (ii) R¹ is ethyl or propyl when combined with R⁴ to form a pyrrolidine ring; and
X is either
(a) =0 ; or
(b) 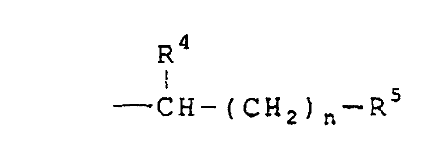
wherein:
R⁴ is selected from the group consisting of methyl and hydrogen and, when combined with R¹, forms a pyrrolidine ring;
R⁵ is selected from the group consisting of -CO₂H and -SO₃H; and
n is an integer of from 0 to 2;
with the proviso that, when R¹ and R⁴ form a pyrrolidine ring, no more than one of R² and R³ is hydrogen, and wherein the compound is added in an amount effective to decrease the incidence of DNA polymerase stops.

2. The method of claim 1 wherein R¹, R² and R³ are the same or different and selected from the group consisting of methyl, ethyl and hydrogen with the proviso that no more than two of R¹, R² and R³ are hydrogen and, when R¹ and R⁴ form a pyrrolidine ring, no more than one of R² and R³ is hydrogen.

3. The method of claim 2 wherein X is -CH₂CO₂H.

4. The method of claim 3 wherein R¹, R² and R³ are methyl.

5. The method of claim 3 wherein R¹, R² are methyl and R³ is hydrogen.

6. The method of claim 3 wherein R¹ is methyl and R² and R³ are hydrogen.

7. The method of claim 2 wherein X is =0.

8. The method of claim 7 wherein R¹, R² and R³ are methyl.

9. The method of claim 2 wherein R¹ and R⁴ form a pyrrolidine ring, R² and R³ are methyl, n is 0, and R⁵ is -CO₂H.

10. The method of claim 2 wherein R¹, R², and R³ are methyl and X is -CH₂SO₃H.

11. The method of claim 1 wherein said reaction mixture is a reaction mixture for a chain termination method of DNA sequencing.

12. The method of claim 11 wherein said chain termination method of DNA sequencing is a dideoxy DNA sequencing method.

13. The method of claim 1 wherein said reaction mixture is a PCR reaction mixture.

14. The method of claim 1 wherein said reaction mixture comprises unpurified DNA.

15. The method of claim 14 wherein said unpurified DNA is a crude cell lysate.

16. A method of decreasing the incidence of DNA polymerase stops in a chain termination DNA sequencing method comprising the steps of:
a) combining in an aqueous solution, a DNA molecule; a DNA polymerase capable of producing a nucleic acid complementary to a portion of said DNA molecule by using the DNA molecule as a template; a mixture of deoxyribonucleoside triphosphates; a chain elongation inhibitor; and a compound of the formula: 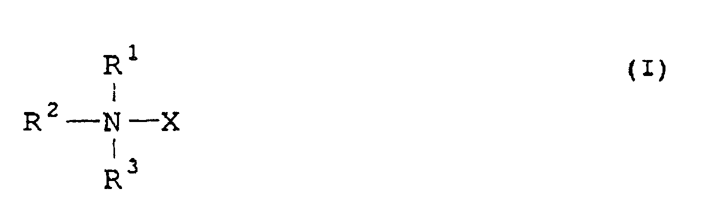 wherein:
R¹, R², and R³ may be the same or different and are independently selected from the group consisting of hydrogen, methyl, ethyl, and propyl, with the provisos that (i) no more than two of R¹, R², and R³ are hydrogen and (ii) R¹ is ethyl or propyl when combined with R⁴ to form a pyrrolidine ring; and
X is either
(a) =0 ; or
(b) 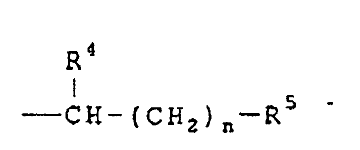
wherein:
R⁴ is selected from the group consisting of methyl and hydrogen and, when combined with R¹, forms a pyrrolidine ring;
R⁵ is selected from the group consisting of -CO₂H and -SO₃H; and
n is an integer of from 0 to 2;
with the proviso that, when R¹ and R⁴ form a pyrrolidine ring, no more than one of R² and R³ is hydrogen, and
wherein the compound is added in an amount effective to decrease the incidence of DNA polymerase stops, to form a reaction mixture; and
b) incubating the reaction mixture to permit the DNA polymerase to form nucleic acid fragments of varying length by using the DNA molecule as a template, wherein said nucleic acid fragments are complementary to said DNA molecule.

17. The method of claim 16 wherein said chain elongation inhibitors are 2',3'-dideoxyribonucleoside triphosphates.

18. The method of claim 16 wherein R¹, R² and R³ are the same or different and selected from the group consisting of methyl, ethyl and hydrogen with the proviso that no more than two of R¹, R² and R³ are hydrogen and, when R¹ and R⁴ form a pyrrolidine ring, no more than one of R² and R³ is hydrogen.

19. The method of claim 18 wherein X is -CH₂CO₂H.

20. The method of claim 19 wherein R¹, R² and R³ are methyl.

21. The method of claim 18 wherein X is =0.

22. The method of claim 21 wherein R¹, R² and R³ are methyl.

23. The method of claim 18 wherein R¹ and R⁴ form a pyrrolidine ring, R² and R³ are methyl, n is 0, and R⁵ is -CO₂H;

24. The method of claim 18 wherein R¹, R², and R³ are methyl and X is -CH₂SO₃H.

25. The method of claim 16 wherein said DNA molecule is unpurified DNA.

26. The method of claim 16 wherein step a) further comprises the addition of a primer complementary to a second portion of said DNA molecule, said second portion of the DNA molecule located downstream to said first portion of the DNA molecule, wherein said DNA polymerase is capable of extending the 3' end of said primer to produce a nucleic acid complementary to said first portion of the DNA molecule.

27. A kit for sequencing DNA by a chain termination method comprising a container which contains one or more deoxyribonucleoside triphosphates, a container which contains a chain elongation inhibitor, and a compound of the formula: 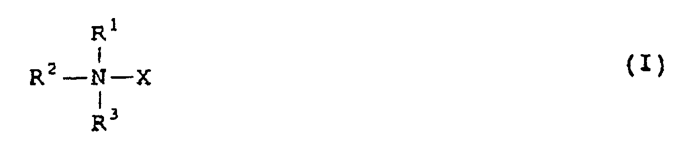 wherein:
R¹, R², and R³ may be the same or different and are independently selected from the group consisting of hydrogen, methyl, ethyl, and propyl, with the provisos that (i) no more than two of R¹, R², and R³ are hydrogen and (ii) R¹ is ethyl or propyl when combined with R⁴ to form a pyrrolidine ring; and
x is either
(a) =0 ; or
(b) 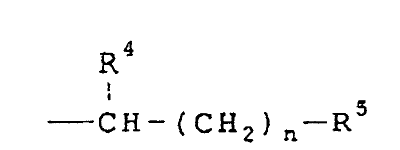
wherein:
R⁴ is selected from the group consisting of methyl and hydrogen and, when combined with R¹, forms a pyrrolidine ring;
R⁵ is selected from the group consisting of -CO₂H and -SO₃H; and
n is an integer of from 0 to 2;
with the proviso that, when R¹ and R⁴ form a pyrrolidine ring, no more than one of R² and R³ is hydrogen.

28. The kit of claim 27 wherein R¹, R² and R³ are the same or different and selected from the group consisting of methyl, ethyl and hydrogen with the proviso that no more two of R¹, R² and R³ are hydrogen and, when R¹ and R⁴ form a pyrrolidine ring, no more than one of R² and R³ is hydrogen.

29. The kit of claim 28 wherein X is -CH₂CO₂H.

30. The kit of claim 29 wherein R¹, R² and R³ are methyl.

31. The kit of claim 28 wherein X is =0.

32. The kit of claim 31 wherein R¹, R² and R³ are methyl.

33. The kit of claim 28 wherein R¹ and R⁴ form a pyrrolidine ring, R² and R³ are methyl, n is 0, and R⁵ is -CO₂H.

34. The kit of claim 28 wherein R¹, R², and R³ are methyl and X is -CH₂-SO₃H.

## Patentansprüche

1. Verfahren, um das Auftreten von DNA-Polymerase-Stopps, die in einem Reaktionsgemisch, das eine DNA-Polymerase enthält, auftreten, zu reduzieren, wobei das Verfahren den Schritt umfasst, dem Reaktionsgemisch eine Verbindung der Formel: 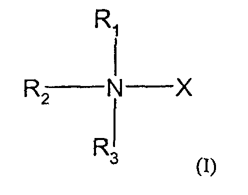 zuzusetzen, wobei:
R₁, R₂, R₃ gleich oder verschieden sein können und unabhängig voneinander ausgewählt sind aus Wasserstoff, Methyl, Ethyl und Propyl, unter der Voraussetzung, dass (i) nicht mehr als zwei von R₁, R₂ und R₃ Wasserstoff sind und (ii) R₁ Ethyl oder Propyl ist, wenn es mit R₄ einen Pyrrolidin-Ring bildet; und X entweder
(a) =O oder
(b) 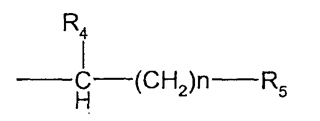 ist, wobei
R₄ ausgewählt ist aus Methyl und Wasserstoff und, wenn es in Kombination mit R₁ ist, einen Pyrrolidin-Ring bildet;
R₅ ausgewählt ist aus -CO₂H und -SO₃H; und
n eine ganze Zahl von 0 bis 2 ist;
unter der Voraussetzung, dass, wenn R₁ und R₄ einen Pyrrolidin-Ring bilden, höchstens eines von R₂ und R₃ Wasserstoff ist,
und wobei die Verbindung in einer Menge zugesetzt wird, die wirksam ist, um das Auftreten von DNA-Polymerase-Stopps zu minimieren.

2. Das Verfahren nach Anspruch 1, wobei R₁, R₂ und R₃ gleich oder verschieden sind und ausgewählt sind aus Methyl, Ethyl und Wasserstoff, unter der Voraussetzung, dass nicht mehr als zwei von R₁, R₂ und R₃ Wasserstoff sind und dass, wenn R₁ und R₄ einen Pyrrolidin-Ring bilden, nicht mehr als eins von R₂ und R₃ Wasserstoff ist.

3. Das Verfahren nach Anspruch 2, wobei X -CH₂CO₂H ist.

4. Das Verfahren nach Anspruch 3, wobei R₁, R₂ und R₃ Methyl sind.

5. Das Verfahren nach Anspruch 3, wobei R₁ und R₂ Methyl sind und R₃ Wasserstoff ist.

6. Das Verfahren nach Anspruch 3, wobei R₁ Methyl ist und R₂ und R₃ Wasserstoff sind.

7. Das Verfahren nach Anspruch 2, wobei X =O ist.

8. Das Verfahren nach Anspruch 7, wobei R₁, R₂ und R₃ Methyl sind.

9. Das Verfahren nach Anspruch 2, wobei R₁ und R₄ einen Pyrrolidin-Ring bilden, R₂ und R₃ Methyl sind, n 0 und R₅ -CO₂H sind.

10. Das Verfahren nach Anspruch 2, wobei R₁, R₂ und R₃ Methyl sind und X -CH₂SO₃H ist.

11. Das Verfahren nach Anspruch 1, wobei das Reaktionsgemisch ein Reaktionsgemisch für ein Kettenabbruchverfahren der DNA-Sequenzierung ist.

12. Das Verfahren nach Anspruch 11, wobei das Kettenabbruchverfahren der DNA-Sequenzierung ein Didesoxy-DNA-Sequenzierungsverfahren ist.

13. Das Verfahren nach Anspruch 1, wobei das Reaktionsgemisch ein PCR-Reaktionsgemisch ist.

14. Das Verfahren nach Anspruch 1, wobei das Reaktionsgemisch ungereinigte DNA enthält.

15. Das Verfahren nach Anspruch 14, wobei die ungereinigte DNA rohes Zell-Lysat ist.

16. Verfahren, um das Auftreten von DNA-Polymerase-Stopps, die in einem Kettenabbruch-DNA-Sequenzierungsverfahren auftreten, zu reduzieren, wobei das Verfahren die Schritte umfasst,
(a) ein DNA-Molekül, eine DNA-Polymerase, die unter Verwendung des DNA-Moleküls als Matrize zur Erzeugung einer Nukleinsäure, komplementär zu einem Teil des DNA-Moleküls, befähigt ist, ein Gemisch aus Desoxyribonukleosid-Triphosphaten, einen Kettenverlängerungsinhibitor und eine Verbindung der Formel: 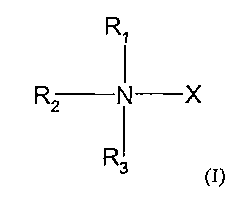 in einer wässrigen Lösung zusammenzugeben, wobei R₁, R₂, R₃ gleich oder verschieden sein können und unabhängig voneinander ausgewählt sind aus Wasserstoff, Methyl, Ethyl und Propyl, unter der Voraussetzung, dass (i) nicht mehr als zwei von R₁, R₂ und R₃ Wasserstoff sind und (ii) R₁ Ethyl oder Propyl ist, wenn es mit R₄ einen Pyrrolidin-Ring bildet; und X entweder
(a) =O oder
(b) 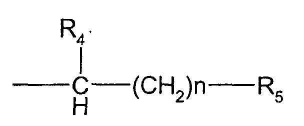 ist, wobei
R₄ ausgewählt ist aus Methyl und Wasserstoff und, wenn es in Kombination mit R₁ ist, einen Pyrrolidin-Ring bildet;
R₅ ausgewählt ist aus -CO₂H und -SO₃H; und
n eine ganze Zahl von 0 bis 2 ist; unter der Voraussetzung, dass, wenn R₁ und R₄ einen Pyrrolidin-Ring bilden, höchstens eines von R₂ und R₃ Wasserstoff ist,
und wobei die Verbindung in einer Menge zugesetzt wird, die wirksam ist, um das Auftreten von DNA-Polymerase-Stopps zu minimieren; und
(b) das Reaktionsgemisch zu inkubieren, um der DNA-Polymerase die Bildung von Nukleinsäurefragmenten verschiedener Länge unter Verwendung des DNA-Moleküls als Matrize zu erlauben, wobei die Nukleinsäurefragmente mit dem DNA-Molekül komplementär sind.

17. Das Verfahren nach Anspruch 16, wobei die Kettenverlängerungsinhibitoren 2',3'-Didesoxyribonukleosid-Triphosphate sind.

18. Das Verfahren nach Anspruch 16, wobei R₁, R₂ und R₃ gleich oder verschieden sind und ausgewählt sind aus Methyl, Ethyl und Wasserstoff, unter der Voraussetzung, dass nicht mehr als zwei von R₁, R₂ und R₃ Wasserstoff sind und, wenn R₁ und R₄ einen Pyrrolidin-Ring bilden, nicht mehr als eins von R₂ und R₃ Wasserstoff ist.

19. Das Verfahren nach Anspruch 18, wobei X -CH₂CO₂H ist.

20. Das Verfahren nach Anspruch 19, wobei R₁, R₂ und R₃ Methyl sind.

21. Das Verfahren nach Anspruch 18, wobei X =O ist.

22. Das Verfahren nach Anspruch 21, wobei R₁, R₂ und R₃ Methyl sind.

23. Das Verfahren nach Anspruch 18, wobei R₁ und R₄ einen Pyrrolidin-Ring bilden, R₂ und R₃ Methyl sind, n 0 und R₅ -CO₂H sind.

24. Das Verfahren nach Anspruch 18, wobei R₁, R₂ und R₃ Methyl sind und X -CH₂SO₃H ist.

25. Das Verfahren nach Anspruch 16, wobei das DNA-Molekül ungereinigte DNA ist.

26. Das Verfahren nach Anspruch 16, wobei Schritt (a) zusätzlich die Zufügung eines Primers komplementär zu einem zweiten Teil des DNA-Moleküls umfasst, wobei der zweite Teil des DNA-Moleküls stromabwärts des ersten Teils des DNA-Moleküls gelegen ist, wobei die DNA-Polymerase fähig ist, das 3'-Ende des Primers unter Herstellung einer Nukleinsäure komplementär dem ersten Teil der DNA auszudehnen.

27. Kit zur Sequenzierung von DNA mittels Kettenabbruchverfahren, umfassend einen Behälter, der mindestens ein Desoxyribonukleosid-Triphosphat enthält, einen Container, der einen Kettenverlängerungsinhibitor enthält und eine Verbindung der Formel 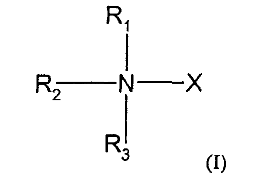 wobei R₁, R₂, R₃ gleich oder verschieden sein können und unabhängig voneinander ausgewählt sind aus Wasserstoff, Methyl, Ethyl und Propyl, unter der Voraussetzung, dass (i) nicht mehr als zwei von R₁, R₂ und R₃ Wasserstoff sind und (ii) R₁ Ethyl oder Propyl ist, wenn es mit R₄ einen Pyrrolidin-Ring bildet; und X entweder
(a) =O oder
(b) 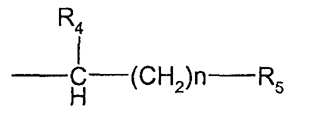 ist, wobei
R₄ ausgewählt ist aus Methyl und Wasserstoff und, wenn es in Kombination mit R₁ ist, einen Pyrrolidin-Ring bildet;
R₅ ausgewählt ist aus -CO₂H und -SO₃H; und
n eine ganze Zahl von 0 bis 2 ist;
unter der Voraussetzung, dass, wenn R₁ und R₄ einen Pyrrolidin-Ring bilden, höchstens eines von R₂ und R₃ Wasserstoff ist.

28. Das Kit nach Anspruch 27, wobei R₁, R₂ und R₃ gleich oder verschieden sind und ausgewählt sind aus Methyl, Ethyl und Wasserstoff, unter der Voraussetzung, dass nicht mehr als zwei von R₁, R₂ und R₃ Wasserstoff sind und dass, wenn R₁ und R₄ einen Pyrrolidin-Ring bilden, nicht mehr als eins von R₂ und R₃ Wasserstoff ist.

29. Das Kit nach Anspruch 28, wobei X -CH₂CO₂H ist.

30. Das Kit nach Anspruch 29, wobei R₁, R₂ und R₃ Methyl sind.

31. Das Kit nach Anspruch 28, wobei X =O ist.

32. Das Kit nach Anspruch 31, wobei R₁, R₂ und R₃ Methyl sind.

33. Das Kit nach Anspruch 28, wobei R₁ und R₄ einen Pyrrolidin-Ring bilden, R₂ und R₃ Methyl sind, n 0 und R₅ -CO₂H sind.

34. Das Kit nach Anspruch 28, wobei R₁, R₂ und R₃ Methyl sind und X -CH₂SO₃H ist.

## Revendications

1. Méthode pour diminuer la fréquence des arrêts de l'ADN polymérase se produisant dans un mélange réactionnel contenant une ADN polymérase comprenant l'addition audit mélange réactionnel d'un composé de formule : 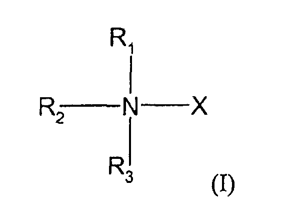 dans laquelle :
R₁, R₂, et R₃ peuvent être identiques ou différents et sont choisis indépendamment dans le groupe consistant en hydrogène, méthyle, éthyle, et propyle, sous réserve que (i) pas plus de deux parmi R₁, R₂, et R₃ sont hydrogène et (ii) R1 est éthyle ou propyle lorsqu'il est combiné avec R₄ pour former un cycle pyrrolidine ; et X est soit
(a) =O ; soit
(b) 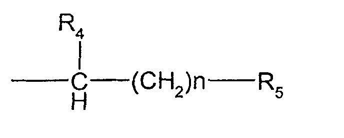
dans lequel :
R₄ est choisi dans le groupe consistant en méthyle et hydrogène et, lorsqu'il est combiné avec R₁, forme un cycle pyrrolidine ;
R₅ est choisi dans le groupe consistant en -CO₂H et SO₂H ;et
n est un nombre entier allant de 0 à 2 ;
sous réserve que lorsque R₁ et R₄ forment un cycle pyrrolidine, pas plus d'un parmi R₂ et R₃ n'est hydrogène, et dans laquelle le composé est ajouté en une quantité efficace pour diminuer la fréquence des arrêts de l'ADN polymérase.

2. Méthode selon la revendication 1 dans laquelle R₁, R₂ et R₃ sont identiques ou différents et sont choisis dans le groupe consistant en méthyle, éthyle et hydrogène sous réserve que pas plus de deux parmi R₁, R₂ et R₃ sont hydrogène et, lorsque R₁ et R₄ forment un cycle pyrrolidine, pas plus d'un parmi R₂ et R₃ est hydrogène.

3. Méthode selon la revendication 2, dans laquelle X est -CH₂CO₂H.

4. Méthode selon la revendication 3, dans laquelle R₁, R₂ et R₃ sont méthyle.

5. Méthode selon la revendication 3, dans laquelle R₁ et R₂ sont méthyle et R₃ est hydrogène.

6. Méthode selon la revendication 3, dans laquelle R₁ est méthyle et R₂ et R₃ sont hydrogène.

7. Méthode selon la revendication 2, dans laquelle X est =O.

8. Méthode selon la revendication 7, dans laquelle R₁, R₂ et R₃ sont méthyle.

9. Méthode selon la revendication 2, dans laquelle R₁ et R₄ forment un cycle pyrrolidine, R₂ et R₃ sont méthyle, n est 0, et R₅ est -CO₂H.

10. Méthode selon la revendication 2, dans laquelle R₁, R₂ et R₃ sont méthyle et X est -CH₂SO₃H.

11. Méthode selon la revendication 1, dans laquelle ledit mélange réactionnel est un mélange réactionnel pour une méthode de séquençage d'ADN par terminaison de chaîne.

12. Méthode selon la revendication 1, dans laquelle ladite méthode séquençage d'ADN par terminaison de chaîne est une méthode de séquençage didéoxy d'ADN.

13. Méthode selon la revendication 1, dans laquelle ledit mélange réactionnel est un mélange réactionnel pour PCR.

14. Méthode selon la revendication 1, dans laquelle ledit mélange réactionnel comprend de l'ADN non purifié.

15. Méthode selon la revendication 14, dans laquelle ledit ADN non purifié est un lysat cellulaire brut.

16. Méthode pour diminuer la fréquence des arrêts de l'ADN polymérase dans une méthode de séquençage d'ADN par terminaison de chaîne comprenant les étapes consistant à:
a) combiner dans une solution aqueuse une molécule d'ADN, une ADN polymérase capable de produire un acide nucléique complémentaire d'une partie de ladite molécule d'ADN par utilisation de la molécule d'ADN en tant que matrice ; un mélange de déoxyribonucléoside triphosphates ; un inhibiteur d'élongation de chaîne ; et un composé de formule : 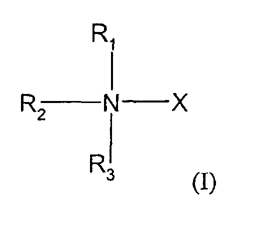 dans laquelle :
R₁, R₂ et R₃ peuvent être identiques ou différents et sont choisis indépendamment dans le groupe consistant en hydrogène, méthyle, éthyle, et propyle, sous réserve que (i)
pas plus de deux parmi R₁, R₂ et R₃ sont hydrogène et (ii) R₁ est éthyle ou propyle lorsqu'il est combiné avec R₄ pour former un cycle pyrrolidine ; et X est soit
(a) =O ; soit
(b) 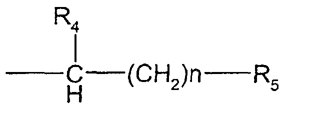
dans lequel :
R₄ est choisi dans le groupe consistant en méthyle et hydrogène et, lorsqu'il est combiné avec R1, forme un cycle pyrrolidine ;
R₅ est choisi dans le groupe consistant en -CO₂H et SO₂H ;et
n est un nombre entier allant de 0 à 2 ;
sous réserve que lorsque R₁ et R₄ forment un cycle pyrrolidine, pas plus d'un parmi R₂ et R₃ est hydrogène, et dans laquelle le composé est ajouté en une quantité efficace pour diminuer la fréquence des arrêts de l'ADN polymérase, pour former un mélange réactionnel ; et
b) mettre le mélange réactionnel à incuber pour permettre à l'ADN polymérase de former des fragments d'acide nucléique de longueur différente en utilisant la molécule d'ADN en tant que matrice, dans laquelle les dits fragments d'acide nucléique sont complémentaires de ladite molécule d'ADN.

17. Méthode selon la revendication 16 ans laquelle lesdits inhibiteurs d'élongation de chaîne sont des 2',3'-didéoxyribonucléoside triphosphates.

18. Méthode selon la revendication 16 dans laquelle R₁, R₂ et R₃ sont identiques ou différents et sont choisis dans le groupe consistant en méthyle, éthyle et hydrogène sous réserve que pas plus de deux parmi R₁, R₂ et R₃ sont hydrogène et, lorsque R₁ et R₄ forment un cycle pyrrolidine, pas plus d'un parmi R₂ et R₃ est hydrogène.

19. Méthode selon la revendication 18, dans laquelle X est -CH₂CO₂H.

20. Méthode selon la revendication 19, dans laquelle R₁, R₂ et R₃ sont méthyle.

21. Méthode selon la revendication 18, dans laquelle X est =O.

22. Méthode selon la revendication 21, dans laquelle R₁, R₂ et R₃ sont méthyle.

23. Méthode selon la revendication 18, dans laquelle R₁ et R₄ forment un cycle pyrrolidine , R₂ et R₃ sont méthyle, n est 0, et R₅ est -CO₂H.

24. Méthode selon la revendication 18, dans laquelle R₁, R₂ et R₃ sont méthyle et X est -CH₂SO₃H.

25. Méthode selon la revendication 16, dans laquelle ladite molécule d'ADN est de l'ADN non purifié.

26. Méthode selon la revendication 16, dans laquelle l'étape a) comprend en outre l'addition d'une amorce complémentaire d'une seconde partie de ladite molécule d'ADN, ladite seconde partie de la molécule d'ADN étant située en aval de ladite première partie de la molécule d'ADN, dans laquelle ladite ADN polymérase est capable d'allonger l'extrémité 3' de ladite amorce pour produire un acide nucléique complémentaire de ladite première partie de la molécule d'ADN.

27. Kit pour le séquençage d'ADN par une méthode de terminaison de chaîne comprenant un récipient qui contient un ou plusieurs déoxyribonucléoside triphosphates, un récipient qui contient un inhibiteur d'élongation de chaîne, et un composé de formule : 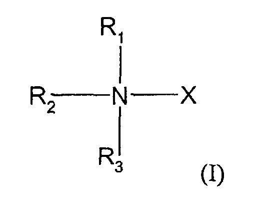 dans laquelle :
R₁, R₂ et R₃ peuvent être identiques ou différents et sont choisis indépendamment dans le groupe consistant en hydrogène, méthyle, éthyle, et propyle, sous réserve que (i) pas plus de deux parmi R₁, R₂ et R₃ sont hydrogène et (ii) R₁ est éthyle ou propyle lorsqu'il est combiné avec R₄ pour former un cycle pyrrolidine ; et X est soit
(a) =O ; soit
(b) 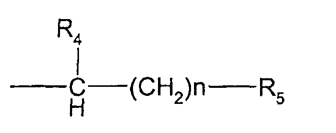
dans lequel :
R₄ est choisi dans le groupe consistant en méthyle et hydrogène et, lorsqu'il est combiné avec R₁, forme un cycle pyrrolidine ;
R₅ est choisi dans le groupe consistant en -CO₂H et SO₂H ;et
n est un nombre entier allant de 0 à 2 ;
sous réserve que lorsque R₁ et R₄ forment un cycle pyrrolidine, pas plus d'un parmi R₂ et R₃ est hydrogène.

28. Kit selon la revendication 27, dans laquelle R₁, R₂ et R₃ sont identiques ou différents et sont choisis dans le groupe consistant en méthyle, éthyle et hydrogène sous réserve que pas plus de deux parmi R₁, R₂ et R₃ sont hydrogène et, lorsque R₁ et R₄ forment un cycle pyrrolidine, pas plus d'un parmi R₂ et R₃ est hydrogène.

29. Kit selon la revendication 28, dans lequel X est -CH₂CO₂H.

30. Kit selon la revendication 29, dans lequel R₁, R₂ et R₃ sont méthyle.

31. Kit selon la revendication 28, dans lequel X est =O.

32. Kit selon la revendication 31, dans lequel R₁, R₂ et R₃ sont méthyle.

33. Kit selon la revendication 28, dans lequel R₁ et R₄ forment un cycle pyrrolidine , R₂ et R₃ sont méthyle, n est 0, et R₅ est -CO₂H.

34. Méthode selon la revendication 28, dans lequel R₁, R₂ et R₃ sont méthyle et X est -CH₂SO₃H.
